# EUROPEAN PATENT SPECIFICATION

(11) **EP 0 519 703 B1**
(45) Date of publication and mention of the grant of the patent: **08.05.1996**
(21) Application number: 92305556.0
(22) Date of filing: 17.06.1992
(51) Int. Cl.: A61B 17/12

(54) **Endoscopic suture clip**
Endoskopische Nahtklammer
Clip de suture endoscopique

(30) Priority: 18.06.1991 US 717297
(43) Date of publication of application: 23.12.1992
(73) Proprietor: ETHICON INC., Somerville New Jersey 08876 (US)
(72) Inventor: Chen, Chao C., Edison, New Jersey 08820 (US); Zwaskis, William, Fanwood, New Jersey 07023 (US); Ortiz, Mark, Milford, Ohio 45150 (US)
(74) Representative: Mercer, Christopher Paul

(56) References cited:
- EP-A- 0 314 064
- US-A- 3 866 611
- US-A- 4 476 865

## Description

### BACKGROUND OF THE INVENTION

This invention relates generally to a surgical clip. More particularly, it relates to such a clip suitably adapted to replace a suture knot during endoscopic surgery.

As medical and hospital costs continue to increase, surgeons are constantly striving to develop advanced surgical techniques. Advances in the surgical field are often related to the development of operative techniques which involve less invasive surgical procedures and reduce overall patient trauma. In this manner, the length of hospital stays can be significantly reduced, and therefore the hospital and medical costs can be reduced as well.

One of the truly great advances in recent years to reduce the invasiveness of surgical procedures is endoscopic surgery. Endoscopic surgery involves the use of an endoscope, which is an instrument permitting the visual inspection and magnification of any cavity of the body. The endoscope is inserted through a cannula after puncture through the wall of the body cavity with a trocar, which is a sharp-pointed instrument. The surgeon can then perform diagnostic and therapeutic procedures at the surgical site with the aid of specialized instrumentation designed to fit through additional cannulas providing small diameter openings into the desired body cavity as may be required.

An age-old procedure which surgeons are required to perform to repair or reconstruct traumatized bodily tissue is suturing. Fortunately, medical instruments have been recently designed to allow a surgeon to manipulate a suture, or suture and needle combination, through the small diameter opening of a cannula. However, the ability to tie an appropriately placed suture knot has become troublesome and problematical.

Therefore, in response to this problem, surgeons have sought alternatives to conventional knot-tying techniques which would be suitable during endoscopic surgery. Among these alternatives include the use of hemostatic clips, which are designed to ligate blood vessels and other tubular members, to replace suture knots. Such hemostatic clips are described, for example, in U.S. Patent Nos. 4,418,694 and 4,476,865 the latter showing the features of the first part of claim 1. These clips can be readily applied with a clip applier which is designed to function through the small opening of a cannula. Unfortunately, the force required to displace these clips from the suture is inadequately low. As a result, hemostatic clips of the type shown in the art are unsuitable for general endoscopic surgery needs.

In view of the deficiencies of the prior art for creating a useful alternative to tying a suture knot, what is desired within the medical community is a device suitable for application using endoscopic techniques which can successfully replace the suture knot. More specifically, what is needed is a clip particularly adapted for replacing a suture knot during endoscopic surgery, and which exhibits adequate clamping force to function effectively.

### BRIEF DESCRIPTION OF THE DRAWINGS

The present invention will be described in greater detail in conjunction with the accompanying drawings wherein:
Figure 1 is a greatly enlarged view in perspective of a clip in accordance with the present invention;
Figure 2 illustrates the clip of Figure 1 clamped about a suture;
Figure 3 illustrates an endoscopic clip applier for endoscopically clamping a suture with a clip of this invention.

### SUMMARY OF THE INVENTION

The invention is an improved surgical clip. The clip is of the type comprising first and second leg members joined at their proximal ends by resilient hinge means and terminating at their distal ends in latch means. Each leg member has an outer surface and a clamping inner surface. The clamping inner surface is disposed in opposition to the clamping inner surface of the other leg member. The outer surface of each leg member is configured to be accepted by the jaws of a clip applier.

The improvement to the clip relates to the dimensions of each leg member. The dimensions of each leg member are defined by a ratio of the length of the clamping inner surface to the width of the leg member of no greater than 1.5.

The clip of this invention is particularly adapted to act as a knot clip in those applications requiring the replacement of a suture knot during endoscopic surgery. The clip, when clamped about a suture, exhibits a clamping force significantly greater than the clamping force exhibited by conventional clips disclosed in the art. This significantly increased clamping force allows the clip to function effectively for the application relating to the replacement of a suture knot. Additionally, the clip can be used for other applications, particularly those applications related to endoscopic surgery.

### DESCRIPTION OF THE PREFERRED EMBODIMENT

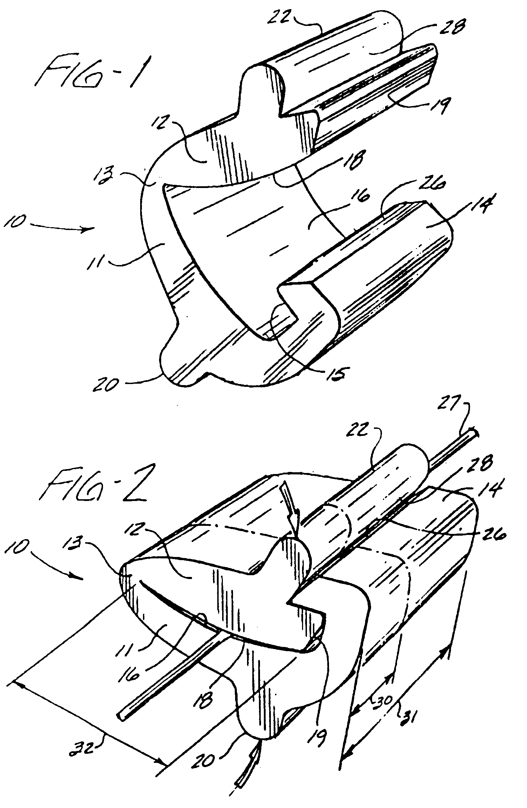

Referring to Figure 1, there is shown a surgical clip 10 of the present invention. The clip has first and second leg members 11 and 12, respectively. The leg members are connected at their proximal ends by a hinge section 13. The hinge section according to the present invention is resilient; i.e., it has elastic memory and acts as a spring which assists in the packaging of the clip as well as the handling and placement of the clip. First leg member 11 terminates at its distal end in a hook member 14. The hook member has an inner face 15. In a preferred embodiment, the end surface of the hook member is beveled at 26 to assist in deflecting the hook member when the clip is closed. The clamping inner surface 16 of the first leg member 11 has a concave radius of curvature extending from the hinge to the start of the hook member. The second leg member 12 has clamping inner surface 18 which has a convex radius of curvature extending from the hinge to the distal end of the leg member. The radius of curvature of the clamping surface 18 is smaller than the radius of curvature of clamping surface 16. Second leg member 12 terminates in an end surface 19. Preferably this end surface is beveled and has a complementary bevel to the bevel on the hook member so as to assist in the deflection of the hook member when the clip is closed.

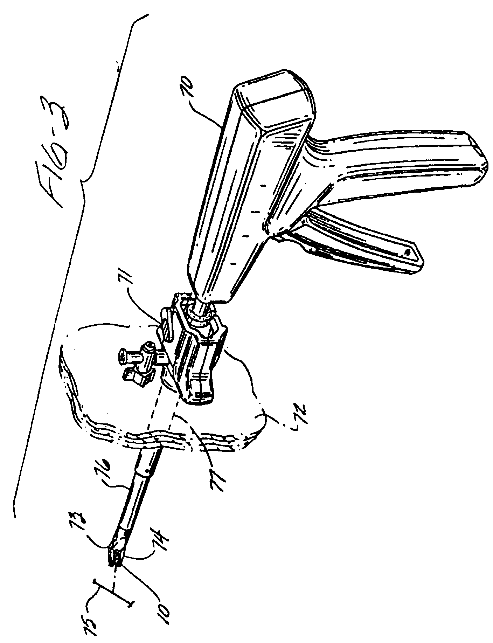

Disposed on the outer surface of each leg member are cylindrical bosses 20 and 22. The bosses are used to manipulate the clip in a suitable instrument, for example, an endoscopic clip applier having a jaw formation suitable for securely grasping the clip, as will be briefly described in conjunction with Figure 3. The clip is closed about a suture as shown in Figure 2 by urging the distal ends of the two leg members together by applying force on cylindrical bosses 20 and 22 in the direction of the arrows until the end surface 19 of first leg member 12 deflects the hook member 14 by engaging end surface 26 of the hook member on first leg 11 as the two leg members are pivoted about the resilient hinge 13 and closed about a suture 27. In this preferred embodiment, cylindrical boss 22 of the second leg member 12 has a contacting face 28 which abuts end surface 26 when the clip is clamped about a suture to ensure adequate and tight closure.

A critical aspect of the clip embodied in Figures 1 and 2 is the width of each leg member 11 and 12 of the clip. The prior art clips suitably designed for hemostasis conventionally have leg members with width 30 as shown in Figure 2, but each leg member 11 and 12 of this invention has a width 31 which is significantly greater than the width of the leg members of conventional clips. For a conventional hemostasis clip with a typical inner clamping surface length, as defined as length 32 in Figure 2, of about 3 mm (120 mil), each leg member will have a width 30 of approximately 1 mm (40 mil). In contrast, width 31 of each leg member for the most preferred clip of this invention as embodied in Figure 2 is approximately 3 mm (120 mil), while the length 32 of the inner clamping surface remains unchanged. The greater width 31 of each leg member increases the length of suture 27 which is securely clamped by the clip, and therefore the pull force required to disengage suture 27 from the clip significantly increases as well. For example, the pull force required to disengage a conventional U.S.P. size 2/0 suture from the clip as measured in an Instron Universal Tester when the clip is clamped about the suture is greater than 0.7 kg (1.5 pounds), preferably greater than 0.9 kg (2.0 pounds). In contrast, clips with a conventional leg member width 30 generally exhibit a pull force for suture disengagement of less than 0.2 kg (0.5 pounds). The clip of this invention is characterized by having a ratio of the length of the inner clamping surface to the width of each leg member of no greater than 1.5. The most preferred clips have such a ratio no greater than 1.2.

The clips within the scope of the present invention are suitably adapted for endoscopic applications to replace a conventional suture knot. This can be accomplished as illustrated in Figure 3. Referring now to Figure 3, there is shown a clip applier 70 having a long, small-diameter longitudinal member 76. Longitudinal member 76 is adapted to be inserted into a conventional trocar 71 through trocar cannula 77. The trocar is used to provide an opening through bodily tissue 72 for access to the surgical site. The clip applier 70 has jaws 73 at its distal end which are configured in such a manner as to facilitate grasping the outer surface of the legs of the clip 74. Clip applier 70 is maneuvered within the surgical site to place clip 74 about suture 75 which is to be clamped.

The clips of the invention can be made of any biocompatible material using conventional fabrication methods. The clips can be composed of various biocompatible metals, e.g. titanium and tantalum, and polymeric materials. Preferably, the clips are made of bioabsorbable polymeric materials such as homopolymers and copolymers of glycolide, lactide and para-dioxanone. The preferred means for fabricating clips from bioabsorbable polymeric materials is to inject a suitable polymer melt into an appropriately designed mold at process conditions conventionally employed for such polymer systems. After the polymer melt cools, the molded polymer shaped in the mold to meet the design criteria of the clip can be readily released from the mold. The molded clip can then be sterilized using conventional methods to render the clip suitable for surgical applications.

The description of this preferred embodiment should not be construed in any way to limit the scope of the claimed invention. Numerous additional embodiments within the scope of the invention will become readily apparent to those skilled in the art.

## Claims

1. A surgical clip comprising first and second leg members (11, 12) joined at their proximal ends by resilient hinge means (13) and terminating at their distal ends in latch means (14, 19, 26), each leg member (11, 12) having an outer surface and a clamping inner surface, said clamping inner surface (16, 18) being in opposition to the clamping inner surface (18, 16) of the other leg member (12, 11), the outer surface of each leg member (11, 12) being configured to be accepted by the jaws of a clip applier (70);
characterised in that the dimensions of each leg member (11, 12) are defined by a ratio of the length (32) of said clamping inner surface (16, 18) to the width (31) of said leg member of no greater than 1.5.

2. The clip of claim 1 wherein the ratio is no greater than 1.2.

3. The clip of claim 1 or claim 2 wherein said first leg member (11) terminates at its distal end thereof with a deflectable hook member (14).

4. The clip of any preceding claim wherein the clamping inner surface (16) of said first leg member (11) has a concave radius of curvature between the hinge means (13) and the hook member (14), and the clamping inner surface (18) of said second leg member (12) has a convex radius of curvature between the hinge means (13) and its distal end, the radius of curvature of the clamping inner surface (18) of said second leg member (12) being smaller than the radius of curvature of the clamping inner surface (16) of said first leg member (11).

5. The clip of any preceding claim wherein the outer surface of each leg member (11, 12) includes a cylindrical boss (20, 22) to facilitate engagement of said clip by the jaws of a clip applier (70).

6. The clip of any preceding claim wherein the width of each leg member (11, 12) is about 3 mm (120 mil).

## Patentansprüche

1. Chirurgische Klammer mit ersten und zweiten Schenkelelementen (11, 12), die an ihren proximalen Enden durch eine elastische Scharniereinrichtung (13) verbunden sind und die an ihren distalen Enden in einer Rasteinrichtung (14, 19, 26) enden, wobei jedes Schenkelelement (11, 12) eine Außenseite und eine klemmende Innenseite aufweist, wobei die eine klemmende Innenseite (16, 18) der klemmenden Innenseite (18, 16) des anderen Schenkelelements (12, 11) gegenüberliegt und die Außenseite jedes Schenkelelements (11, 12) so gestaltet ist, daß sie von den Backen eines Klammeranlegers (70) aufgenommen wird;
dadurch **gekennzeichnet**, daß die Abmessungen jedes Schenkelelements (11, 12) durch ein Verhältnis der Länge (32) der klemmenden Innenseite (16, 18) zur Breite (31) des Schenkelelements von nicht mehr als 1,5 bestimmt sind.

2. Klammer nach Anspruch 1, wobei das Verhältnis nicht größer als 1,2 ist.

3. Klammer nach Anspruch 1 oder Anspruch 2, wobei das erste Schenkelelement (11) an seinem distalen Ende in einem auslenkbaren Hakenelement (14) endet.

4. Klammer nach einem der vorstehenden Ansprüche, wobei die klemmende Innenseite (16) des ersten Schenkelelements (11) zwischen der Scharniereinrichtung (13) und der Hakeneinrichtung (14) einen konkaven Krümmungsradius und die klemmende Innenseite (18) des zweiten Schenkelelements (12) zwischen der Scharniereinrichtung (13) und seinem distalen Ende einen konvexen Krümmungsradius aufweist, wobei der Krümmungsradius der klemmenden Innenseite (18) des zweiten Schenkelelements (12) kleiner ist als der Krümmungsradius der klemmenden Innenseite (16) des ersten Schenkelelements (11).

5. Klammer nach einem der vorstehenden Ansprüche, wobei die Außenseite der Schenkelelemente (11, 12) einen zylindrischen Wulst (20, 22) zum Erleichtern des Eingriffs der Klammer mit den Backen eines Klammeranlegers (70) aufweist.

6. Klammer nach einem der vorstehenden Ansprüche, wobei die Breite der Schenkelelemente (11, 12) etwa 3 mm (120 mil) betragt.

## Revendications

1. Pince chirurgicale avec une première et une seconde branche (11, 12) réunies à leurs extrémités proximales par une charnière élastique (13) et se terminant à leurs extrémités distales par des moyens de verrouillage (14, 19, 26), chaque branche (11, 12) comportant une surface extérieure et une surface intérieure de serrage, cette surface intérieure de serrage (16, 18) faisant face à la surface intérieure de serrage (18, 16) de l'autre branche (12, 11) et la surface extérieure de chaque branche (11, 12) étant conformée de manière à pouvoir s'insérer dans les mâchoires d'un outil (70) à appliquer les pinces,
caractérisée en ce que les dimensions de chaque branche (11, 12) sont déterminées par un rapport de la longueur (32) de la surface intérieure de serrage (16, 18) à la largeur (31) de la branche ne dépassant pas 1,5.

2. Pince selon la revendication 1, caractérisée en ce que le rapport ne dépasse pas 1,2.

3. Pince selon la revendication 1 ou la revendication 2, caractérisée en ce que la première branche (11) se termine à son extrémité distale par un crochet (14) pouvant être basculé.

4. Pince selon une des revendications précédentes, caractérisée en ce que la surface intérieure de serrage (16) de la première branche (11) présente un rayon de courbure concave entre la charnière (13) et le crochet (14), et que la surface intérieure de serrage (18) de la seconde branche (12) présente un rayon de courbure convexe entre la charnière (13) et son extrémité distale, le rayon de courbure de la surface intérieure de serrage (18) de la seconde branche (12) étant inférieur à celui de la surface intérieure de serrage (16) de la première branche (11).

5. Pince selon une des revendications précédentes, caractérisée en ce que la surface extérieure de chaque branche (11, 12) comporte une bosse cylindrique (20, 22) facilitant la prise de la pince par les mâchoires d'un outil (70) à appliquer les pinces.

6. Pince selon une des revendications précédentes, caractérisée en ce que la largeur de chaque branche (11, 12) est d'environ 3 mm (120 mil).
